# EUROPEAN PATENT SPECIFICATION

(11) **EP 3 813 812 B1**
(45) Date of publication and mention of the grant of the patent: **03.12.2025**
(21) Application number: 19797109.6
(22) Date of filing: 03.05.2019
(51) Int. Cl.: A61K 31/167, C12Q 1/34, G01N 21/78, G01N 33/52, G01N 33/53, G01N 33/58

(54) **ACETAMINOPHEN ASSAY**
ACETAMINOPHENASSAY
DOSAGE D'ACÉTAMINOPHÈNE

(30) Priority: 03.05.2018 US 201862666282 P
(43) Date of publication of application: 05.05.2021
(73) Proprietor: Sekisui Diagnostics, LLC, Burlington, MA 01803 (US)
(72) Inventor: ACORN, Robert, Charlottetown, PE, C1E 2B9 (CA); COADY, Helen, Charlottetown, PE, C1E 2B9 (CA); COLL, Graham, Charlottetown, PE, C1E 2B9 (CA)
(74) Representative: Maschio & Soames IP Ltd
(86) International application number: PCT/US2019/030558
(87) International publication number: WO 2019/213489

(56) References cited:
- EP-A2- 0 750 197
- US-A- 4 224 034
- US-A- 415 929
- US-A- 5 151 370
- US-A1- 2009 269 792
- US-A1- 2009 269 792
- LAWRENCE H KEITH ET AL: "Passage", 9 December 1991, NATIONAL TOXICOLOGY PROGRAM'S CHEMICAL SOLUBILITY COMPENDIUM, CRC PRESS, US, PAGE(S) 1 - 10, ISBN: 978-0-87371-653-6, XP009524588
- GENZYME DIAGNOSTICS P.E.I INC.: "Acetaminophen L3K Assay", 1 May 2009 (2009-05-01), pages 1 - 6, XP055758612, Retrieved from the Internet <URL:https://www.accessdata.fda.gov/cdrh_docs/pdf8/K081938.pdf> [retrieved on 20201210]
- "National Toxicology Program's Chemical Solubility Compendium", 9 December 1991, CRC PRESS, US, ISBN: 978-0-87371-653-6, article LAWRENCE H KEITH; DOUGLAS B WALTERS: "Passage", pages: 1 - 10, XP009524588
- GENZYME DIAGNOSTICS P.E.I INC., ACETAMINOPHEN L3K ASSAY, 1 May 2009 (2009-05-01), pages 1 - 6, XP055758612, Retrieved from the Internet <URL:https://www.accessdata.fda.gov/cdrh_docs/pdf8/K081938.pdf> [retrieved on 20190611]
- SEKISUI DIAGNOSTICS PEI INC.: "SEKURE Acetaminophen L3K Assay", FDA, 8 February 2019 (2019-02-08), pages 1 - 13, XP055649731, Retrieved from the Internet <URL:https://fda.report/PMN/K180835/18/K180835.pdf> [retrieved on 20190611]

## Description

### FIELD OF THE INVENTION

In general, the present invention relates to an assay for determining the concentration of p-aminophenol present in a sample. More particularly, the present invention relates to an improvement of an enzyme-based assay for determining the concentration of acetaminophen present in a sample.

### BACKGROUND OF THE INVENTION

Drug toxicity is a leading cause of acute liver failure. In the evaluation of hepatic failure, the clinical laboratory plays a vital role in diagnosis so that appropriate treatment can be initiated in a timely manner.

Acetaminophen (N-acetyl-p-aminophenol) has long been prescribed as an analgesic and antipyretic. It is widely available without prescription and is an active component in many common therapeutic formulations, such as cold and flu remedies. The widespread use of this drug places it high on the list of suspected hepatotoxic agents in patients presenting with liver malfunction.

While therapeutic doses of acetaminophen rarely cause adverse effects, cases have been reported where chronic, excessive use of acetaminophen has led to hepatotoxicity and nephrotoxicity. Ingestion of acute overdose quantities of acetaminophen causes a depletion of glutathione stores and accumulation of toxic metabolites in the liver, which can cause severe or even fatal liver failure.

When acetaminophen is ingested in excessive quantities, a highly reactive intermediate, N-acetyl-p-benzoquinoneimine, accumulates in the liver. This intermediate reacts with thiols in the liver, particularly glutathione. Glutathione is oxidized to glutathione disulfide (GSSG).

Excessive levels of GSSG in the liver cause necrosis. Acetaminophen toxicity is generally reported at serum concentrations above about 20 mg/dL (1324 µmol/L).

The glutathione precursor, N-acetylcysteine (NAC), is often administered as an antidote for acetaminophen overdose. About 70% of NAC administered is metabolized in the liver. It is believed that NAC functions as an antidote for at least the following reasons: it is a precursor for glutathione, it is a powerful anti-oxidant, and it increases the efficiency of GSSG reductase in the liver. The administration of NAC is believed to minimize or prevent the damage caused by an overdose of acetaminophen, at least in part, by replenishing glutathione stores and preventing an accumulation of GSSG in the liver.

A high concentration of NAC is often administered in an initial loading dose followed by maintenance levels of NAC throughout the course of treatment. The loading dose can result in serum levels of NAC of 2000 mg/L or higher, and maintenance levels are often about 800 mg/L to 1000 mg/L. It is desirable to monitor acetaminophen levels throughout the course of NAC treatmentto ensure an appropriate therapeutic level is maintained while avoiding unnecessary or excessive exposure to NAC.

The incidence of accidental, as well as intentional, acetaminophen overdose has increased significantly. The diagnosis and treatment of acetaminophen overdose requires early detection and accurate measurement of the drug in the body. The amount of acetaminophen on board must be quickly and accurately determined so that clinicians can rapidly administer an appropriate therapeutic dose of NAC to the patient. There is a high demand for rapid, reliable and robust clinical assays for determining acetaminophen concentration in biological samples.

Known methods for determining acetaminophen levels in biological samples include, for example, various chromatographic and spectrophotometric techniques.

Gas-liquid chromatography and high-performance liquid chromatography have proven to be reliable and accurate methods for determining acetaminophen levels in biological samples; however both are lengthy procedures that require expensive instrumentation and a high level of technical skill to perform. Such methods are not particularly suited for Stat laboratories, where rapid results are required.

Differential spectrophotometry has been widely used but this method requires time-consuming solvent extractions, which are undesirable in clinical assays. More rapid spectrophotometric methods generally fail to offer the desired specificity.

Colorimetric techniques include simple colorimetry as well as enzyme-based colorimetric assays. Various immuno-based assays are also available but these tend to be significantly more expensive and therefore less desirable, particularly in a clinical setting.

While enzyme-based assays are convenient and economical compared to immuno-based assays, they are generally less reliable in that they are prone to interference with biological molecules often present in patient samples, such as bilirubin and hemoglobin. Elevated levels of such molecules in patient samples can cause false positive results (see, for example, Bertholf et al., 2003), which can potentially lead to misdiagnosis and inappropriate choice or dose of treatment.

Known enzymatic assays are also subject to interference in the presence of therapeutic levels of NAC. Therefore, enzymatic assays cannot generally be used to monitor acetaminophen levels during the course of NAC treatment due to inaccuracy in the acetaminophen levels measured. This is a significant disadvantage of known enzymatic acetaminophen assays.

Known enzymatic assays employ three main components: an aryl acylamidase enzyme, a chromogenic (or color-forming) compound, and an oxidizing agent of sufficient oxidative potential to catalyze the coupling reaction.

Aryl acylamidase cleaves the amide bond of acetaminophen to yield p-aminophenol and acetate. The p-aminophenol is then reacted with the chromogenic compound in an oxidative coupling reaction in the presence of an oxidizing catalyst to form a colored product. Typical catalysts include metal salts or metal complexes of species having reactive oxygen or functional groups, such as permanganate, periodate persulfate, sulfate, or acetate. The change in absorbance, typically measured at a wavelength that captures the peak absorbance of the colored product, is then used to determine the concentration of acetaminophen in the sample. This may be determined by comparing the absorbance values obtained against a standard or set of standards having known acetaminophen concentration and assayed by the same method. The number of moles of colored product formed is typically proportional to the number of moles of acetaminophen initially present in the sample.

The earliest enzyme-based acetaminophen assays required very long incubation times, often greater than 1 hour for each of the hydrolysis and oxidative coupling reactions, thus rendering them unsuitable for use in an emergency clinical setting.

Hammond et al. (1984) developed a rapid enzyme-based assay for determining acetaminophen concentration in serum using an aryl acylamidase to hydrolyze acetaminophen to p-aminophenol. The p-aminophenol is subsequently reacted with o-cresol in an oxidative coupling reaction catalyzed by copper sulfate, to form an indophenol dye. The change in absorbance at the peak wavelength of the dye (615 nm) is then used to determine acetaminophen levels. While this method provides rapid detection of acetaminophen, it is subject to significant interference in the presence of therapeutic levels of NAC and cannot be used reliably during NAC treatment. A similar method utilizing o-cresol in the presence of an oxidizing catalyst is prone to bilirubin interference (Bertholf et al., 2003), leading to false positive results in hyperbilirubinemic patients.

Morris et al. (1990) disclose an automated enzyme-based assay for measuring acetaminophen in a sample. Automated assays are generally preferred for clinical laboratories. The method uses an aryl acylamidase for hydrolysis of acetaminophen to p-aminophenol, followed by oxidative coupling with 8-hydroxyquinoline in the presence of manganese ions to form a blue product. The reagents are lyophilized for storage stability and must be reconstituted prior to use. Assays involving a reconstitution step are less desirable than liquid-stable assays and are more prone to error.

Known acetaminophen assays using 8-hydroxyquinoline or a derivative thereof as a chromophore are subject to interference in the presence of therapeutic levels of NAC (i.e. >800 mg/L). Bulger et al (US 8,236,519 B2) disclose testing two commercially available acetaminophen assays (Sekisui Diagnostics P.E.I. Inc., PEI, Canada) containing either 8-hydroxyquinoline-5-sulfonic acid (8-HQ5SA) or 8-hydroxyquinoline hemisulfate (8-HQHS) as the chromophore. Although accurate acetaminophen measurements in the absence of NAC were seen, there was a significant (i.e. >about 10%) decrease in acetaminophen recovery in the presence of therapeutic levels of NAC. It was discovered that the presence of NAC affected the oxidative coupling reaction in the assay rather than the enzymatic conversion of acetaminophen to p-aminophenol. There was a considerable difference in recovery between the 8-HQ5SA and 8-HQHS assays, with the 8-HQ5SA assay being significantly more susceptible to NAC interference, indicating that even a slight difference in the chemical structure of the chromophore can be crucial to the coupling reaction when NAC is present

Chen et at (2004) describe an assay for quantifying p-aminophenol in urine to assess exposure to aniline in the workplace. Urine p-aminophenol levels serve as a biological marker of aniline toxicity since about 15 to 60% of absorbed aniline is oxidized to p-aminophenol in vivo. The urine must be acidified and pretreated to release free p-aminophenol from the conjugated forms excreted in urine. The assay involves an oxidative coupling reaction using 2,5-dimethylphenol (p-xylenol) as the chromophore to form a colored product. The coupling reaction is catalyzed by sodium periodate, a strong oxidizer, to form a colored product. It was speculated that quantifying p-aminophenol levels in urine may be useful for assessing acetaminophen overdose, although this was neither explored nor demonstrated.

Afshari and Lui (2001) describe a non-enzymatic method for quantification of acetaminophen in serum. Free unconjugated acetaminophen is first separated from endogenous interferents by an extraction step followed by hydrolysis to p-aminophenol using heat (i.e. boiling for 10 minutes) and acid. This is a non-selective hydrolysis reaction compared to an enzymatic reaction. The hydrolysis reaction is followed by oxidative coupling of p-aminophenol to 2,5-dimethylphenol (p-xylenol) in the presence of sodium periodate, a strong oxidizer, to form a colored product. The need to extract the acetaminophen from the sample and boil the samples renders this method undesirable for use in an emergency clinical setting and also unsuitable for automation.

While enzymatic acetaminophen assays are convenient and more affordable than immuno-based assays, many clinical laboratories favor the immuno-based assays since they are unaffected by the presence of NAC in a sample. It is desirable to reliably measure acetaminophen levels during the course of NAC treatment. Immuno-based assays are also less susceptible to interference in the presence of biological molecules, such as bilirubin and hemoglobin, often present in patient samples. Since serum levels of bilirubin and hemoglobin are not predictable from patient to patient, an assay that is prone to interference with these molecules will not provide a robust clinical test that is reliable for all patients.

Accordingly, Bulger et al (US 8,236,519 B2) disclose a rapid acetaminophen assay that is accurate and reliable in the presence or absence of NAC, and which is less expensive than conventional immuno-based assays. The assay disclosed by Bulger et al (US 8,236,519 B2) is also less susceptible to interference with biological molecules present in patient samples, such as bilirubin, or hemoglobin compared to known assays.

It is well known in the art that the presence of lipid molecules (Lipemia) is one of the most common pre-analytical interferences, the interference resulting from sample turbidity caused by accumulation of the lipoprotein particles. Lipemia can cause increased absorption of light, affecting tests that use spectrophotometric methods.

It is therefore desirable to provide a rapid acetaminophen assay that is accurate and reliable in the presence or absence of NAC, and which is less expensive than conventional immuno-based assays, and which is also less susceptible to interference with lipid molecules present in patient samples, compared to known assays.

### SUMMARY OF THE INVENTION

In a first aspect, the present invention provides a method for determining the concentration of acetaminophen in an aqueous sample, the assay comprising the steps of:
contacting the aqueous sample with a first reagent (Rl) comprising an aryl acylamidase enzyme and a suitable diluent to form a hydrolysis solution, optionally, diluting the hydrolysis solution;
incubating the hydrolysis solution to permit a hydrolysis reaction wherein the acetaminophen is converted to p-aminophenol;
contacting the hydrolysis solution with a second reagent (R2) comprising a xylenol chromophore where the xylenol chromophore is pre-dissolved in DMSO (Dimethyl sulfoxide), to form an oxidative coupling solution;
incubating the oxidative coupling solution to permit an oxidative coupling reaction wherein the xylenol chromophore is coupled to the p-aminophenol in the presence of a suitable catalyst to form a colored product; and
determining the amount of the colored product formed, the amount of the colored product formed being proportional to the amount of acetaminophen present in the aqueous sample,
wherein the xylenol chromophore is selected from the group consisting of 2,5-dimethylphenol, 2,6-dimethylphenol and 2,3-dimethylphenol and wherein the catalyst is a hydrated MnCl₂, and
wherein the method is reliable in the presence or absence of therapeutic levels of N-acetyl cysteine (NAC) in the aqueous sample.

The assay has an advantage over the prior art in that it provides accurate and reliable results in the presence or absence of NAC and can therefore be used to measure acetaminophen levels during NAC treatment. In certain embodiments, the assay has the additional advantage of improved performance and reduced interference with biological molecules compared to known assays. In particular, the assay has the additional advantage of improved performance and reduced interference with lipid molecules in the presence or absence of NAC compared to known assays.

It has been surprisingly discovered that the choice of a xylenol compound selected from the group consisting of 2,5-dimethylphenol, 2,6-dimethylphenol and 2,3-dimethylphenol as a chromophore in an oxidative coupling reaction with p-aminophenol results in improved accuracy and reduced interference in the presence of NAC compared to other known chromophores. Further, it has unexpectedly been discovered that the use of Dimethyl sulfoxide (DMSO) as a solvent for the xylenol chromophore results in improved accuracy and reduced interference with lipid molecules in the presence of NAC as compared to the use of water or water based solvents.

The amount of the colored product formed is proportional to the amount acetaminophen initially present in the aqueous sample. The method is suitable for use in the presence or absence of therapeutic levels of N-acetylcysteine (NAC) in the aqueous sample, and in the presence or absence of other additional interferents such as lipid molecules.

In another aspect, the invention provides for a kit for determining the concentration of acetaminophen in a sample, the kit comprising: a first reagent (R1) comprising an aryl acylamidase for hydrolyzing acetaminophen to p-aminophenol; a second reagent (R2) comprising a xylenol chromophore for oxidative coupling to the p-aminophenol; and a suitable catalyst for catalyzing the oxidative coupling of the xylenol chromophore and the p-aminophenol, wherein the xylenol chromophore is 2,5-dimethylphenol and the catalyst is hydrated MnCl₂, and wherein R2 comprises 2,5-dimethylphenol dissolved in DMSO. In another aspect of the kit, the sample is serum or plasma.

In an embodiment of the kit, the reagents are liquid-stable. In another embodiment of the kit, the kit further comprises instructions for carrying out an acetaminophen determination assay. In one aspect, the instructions set forth the steps of: contacting the sample with R1 and a first suitable diluent to form a hydrolysis solution; incubating the hydrolysis solution to permit a hydrolysis reaction, wherein acetaminophen in the sample is converted to p-aminophenol; contacting the hydrolysis solution with R2 and optionally a second suitable diluent, to form an oxidative coupling solution, where R2 comprises 2,5-dimethylphenol and DMSO; incubating the oxidative coupling solution to permit an oxidative coupling reaction, wherein the xylenol chromophore is coupled to the p-aminophenol in the presence of the catalyst to form a colored product; and determining the amount of the colored product formed, wherein the amount of the colored product is proportional to the amount of acetaminophen initially present in the sample.

In an embodiment of the kit, the assay is reliable in the presence of i) biological molecules present in biological fluids, or ii) therapeutic levels of N-acetylcysteine (NAC). In another aspect of the kit, the biological molecules are selected from the group consisting essentially of bilirubin and hemoglobin. In another aspect of the kit, the therapeutic levels of NAC are greater than 800 mg/L. In another aspect of the kit, R1 comprises aryl acylamidase at a concentration of about 10 U/L to about 5000 U/L. In another aspect of the kit, R2 comprises xylenol chromophore at a concentration of about 0.075 g/L to about 115 g/L. In yet another aspect of the kit, the catalyst is present in R1 in a concentration of about 0.0005 g/L to about 1.000 g/L. In another aspect of the kit, R2 comprises 2,5-dimethylphenol in a concentration of about 0.075 g/L to about 115 g/L and the catalyst is MnCl2.4H2O and is present in R1 in a concentration of about 2.5 g/L to about 20 g/L. In another aspect of the kit, R2 further comprises reduced glutathione in a concentration of about 0.005 g/L to about 5.000 g/L. In yet another aspect of the kit, R1 further comprises a protein solubilizer, a protein stabilizer, an enzyme stabilizer, a metal chelator, a buffer, a surfactant, a pH adjuster, a preservative, an excipient, or a combination thereof.

In an embodiment of the kit, the enzyme stabilizer is selected from one or more of the group consisting essentially of polyvinylpyrrolidone 40,000 MW, BSA Fraction V, trehalose, sodium p-hydroxybenzoate, p-hydroxybenzoic acid, and combinations thereof.

In another aspect of the kit, R2 further comprises one or more of a buffer, a surfactant, a pH adjuster, a preservative, an antioxidant and an excipient. In yet another aspect of the kit, R1 comprises about 932.7 U/L aryl acylamidase and about 0.0525 g/L MnCl2.4H2O; and wherein R2 comprises about 3.75 g/L 2,5-dimethylphenol and about 0.5 g/L reduced glutathione.

In an embodiment of the assay, R1 comprises aryl acylamidase at a concentration of about 10 U/L to about 5000 U/L. In an embodiment of the assay, the xylenol chromophore is 2,5-dimethylphenol. In an embodiment of the assay, the catalyst is a weak oxidizing catalyst and wherein the catalyst is present in R1 in a concentration of about 0.0005 g/L to about 1.000 g/L.

In an embodiment of the assay, R2 comprises 2,5-dimethylphenol in a concentration of about 0.075 g/L to about 115 g/L and/or wherein the catalyst is MnCl₂.4H2O and is present in R1 in a concentration of about 2.5 g/L to about 20 g/L. In an embodiment of the assay, R2 further comprises reduced glutathione in a concentration of about 0.005 g/L to about 5.000 g/L. In an embodiment of the assay, R1 further comprises one or more of a protein solubilizer, a protein stabilizer, an enzyme stabilizer, a metal chelator, a buffer, a surfactant, a pH adjuster, a preservative, or an excipient. In an embodiment of the assay, the enzyme stabilizer is selected from the group consisting of PVP-40, BSA Fraction V, trehalose, sodium p-hydroxybenzoate, p-hydroxybenzoic acid and combinations thereof. In an embodiment of the assay, R2 further comprises one or more of a buffer, a surfactant, a pH adjuster, a preservative, an antioxidant or an excipient. In an embodiment of the assay, the diluent is deionized water and the hydrolysis solution is diluted approximately 1: 1 with the diluent prior to the hydrolysis reaction.

In an embodiment of the assay, R1 comprises about 932.7 U/L aryl acylamidase and about 0.0525 g/L MnCl₂.4H2O; and R2 comprises about 7.5 g/L 2,5-dimethylphenol and about 0.500 g/L reduced glutathione. In an embodiment of the assay, the hydrolysis reaction and the oxidative coupling reaction each take place at a temperature of about 37° C for about 3 to 10 minutes, and the hydrolysis reaction takes place at a pH of about 8.6 and the oxidative coupling reaction takes place at a pH of about 10.8.

In an aspect of the assay, the concentration of acetaminophen is determined by obtaining the difference in absorbance at the end of the hydrolysis reaction and at the end of the oxidative coupling reaction; and comparing the difference against a standard or set of standards, wherein the absorbance is measured at a wavelength between about 610 nm and 665 nm. In an embodiment of the assay, the absorbance is measured at a wavelength of about 660 nm. In an embodiment of the assay, the aqueous sample is serum or plasma.

### DETAILED DESCRIPTION OF THE INVENTION

In general, the present invention relates to a reliable assay for the quantitative determination of p-aminophenol in a sample. More particularly, the present invention relates to an enzyme-based assay for the quantitative determination of acetaminophen in a sample. The assay has an advantage over the prior art in that it provides accurate and reliable results in the presence or absence of NAC and in the presence or absence of other additional interferents such as lipid molecules. In certain embodiments, the assay has the additional advantage of reduced interference with biological molecules compared to known assays.

The samples to be tested are preferably aqueous samples, meaning that they have an aqueous base component. Exemplary aqueous samples which may be tested in the assay include, but are not limited to, water, whole blood, plasma, serum, lymph, bile, urine, spinal fluid, sputum, saliva, perspiration, stool secretions, and the like. It is also possible to assay fluid preparations of human or animal tissue, such as skeletal muscle, heart, kidney, lung, brain, bone marrow, skin, and the like. Exemplary fluid preparations include tissue homogenates and supernatants thereof.

In one embodiment, the aqueous sample to be tested is plasma, serum, or urine. In another embodiment, the aqueous sample is plasma or serum. In one embodiment, the aqueous sample is serum.

The assay of the present invention is carried out in two parts. The first part involves the enzymatic hydrolysis of acetaminophen to p-aminophenol. The second part involves the oxidative coupling of p-aminophenol to a xylenol chromophore in the presence of an appropriate catalyst to form a colored product. In some embodiments, the preferred catalyst is selected from weak oxidizers. The concentration of acetaminophen in the sample may then be determined, for instance, by measuring the change in absorbance at a given wavelength and comparing the value obtained against a standard or set of standards having a known acetaminophen concentration.

In one embodiment, the assay is a two-part assay carried out as follows.

In the first part, an aliquot of sample is brought into contact with a first reagent (R1) containing the enzyme to form a hydrolysis solution. This first reagent may be referred to as the enzyme reagent. The hydrolysis solution containing the sample and R1 is mixed and optionally diluted. In one embodiment, the optional dilution step involves a 1:1 dilution of R1 with a suitable diluent, such as deionized water. The solution is mixed and the hydrolysis reaction is continued to completion at an appropriate temperature to allow the hydrolysis of acetaminophen in the sample to p-aminophenol. An absorbance value is obtained at a given wavelength.

In the second part, upon completion of hydrolysis, a second reagent (R2) containing the xylenol chromophore dissolved in DMSO, is added to the hydrolysis solution and the resulting mixture is briefly mixed. The second reagent (R2) may be referred to as the chromophore reagent. Oxidative coupling of the xylenol chromophore with p-aminophenol requires the presence of a suitable catalyst. In a preferred embodiment, the catalyst for the oxidative coupling reaction is a component of R1 such that the catalyst and chromophore do not associate until R1 and R2 are combined. Alternatively, the catalyst may be a component of R2, or may be added to the mixture of R1 and R2 to drive the oxidative coupling step. The oxidative coupling reaction is continued to completion at an appropriate temperature. Upon completion, absorbance is measured at a given wavelength ranging from approximately 600 nm to approximately 665 nm, and the change in absorbance between the first part and the second part is calculated.

To determine the amount of acetaminophen initially present in the sample, the change in absorbance is compared against a standard, or a set of standards, prepared by the same method and using known concentrations of acetaminophen. Dilution factors must be accounted for. Such calculations are routine to those skilled in the art.

This two-part assay is suitable for automation since no extraction or separation steps are required and only two reagents are utilized. On board dilution and mixing steps can also be carried out in an automated fashion. Automated instruments for carrying out such assays are well known in the art. Alternatively, the assay may be conducted manually.

The enzyme for the hydrolysis reaction is an aryl acylamidase enzyme. Aryl acylamidase enzymes catalyze the hydrolysis of anilides to anilines, and are identified by the IUB (International Union of Biochemistry) number E.C.3.5.1.13. The CAS registry number for this class of enzymes is 9025-18-7. Aryl acylamidase enzymes are typically produced by and isolated from microorganisms, such as bacteria. Non-limiting examples of aryl acylamidase enzymes and methods of producing them from microorganisms are described, for example, in U.S. Pat. No. 4,430,433 to Hammond et al.

Any suitable aryl acylamidase enzyme may be used in accordance with the present invention so long as it is able to effectively catalyze the hydrolysis of acetaminophen to p-aminophenol under appropriate reaction conditions. The reaction conditions may be optimized by a person skilled in the art in view of the particular enzyme selected without departing from the present invention.

The aryl acylamidase may be present in any suitable amount. The aryl acylamidase is preferably present in a sufficient concentration such that substantially all of the acetaminophen present in a sample will be converted to p-aminophenol. In one embodiment, R1 comprises aryl acylamidase at a concentration of about 10 U/L to about 5000 U/L, or about 600 U/L to about 1200 U/L, or about 800 U/L to about 1000 U/L. In one embodiment, R1 comprises aryl acylamidase at a concentration of about 932.7 U/L.

The solvent or diluent for R1 may be any suitable aqueous-based solvent or diluent that does not negatively impact the assay. In one embodiment, the solvent or diluent is water, preferably distilled water, deionized water, or reverse osmosis water. In one embodiment, the diluent is deionized water. The solvent or diluent may comprise various additives and components.

In addition to the aryl acylamidase, R1 may further comprise one or more of a catalyst, a cofactor, a protein solubilizer, a protein stabilizer, an enzyme stabilizer, a metal chelator, a buffer, a surfactant, a pH adjuster, a preservative, a diluent, a solvent, an excipient or the like.

In one embodiment, R1 comprises the catalyst for the oxidative coupling reaction. Any suitable catalyst may be utilized in accordance with the invention, in any suitable concentration, if is capable of sufficiently catalyzing the oxidative coupling reaction. Exemplary catalysts include, but are not limited to, permanganates, periodates, persulfates, acetates, and other metal salts. In one embodiment, the catalyst is a metal salt selected from FeCl₃, MnCl₂, CuSO₄, KIO₄ or a derivative thereof. In a preferred embodiment, the catalyst is a weak oxidizer. In one embodiment, the weak oxidizing catalyst is manganese (II) chloride, MnCl₂. In one embodiment, the catalyst is manganese (II) chloride tetrahydrate, MnCl₂.4H₂O.

In certain embodiments, the catalyst is present in R1 in a concentration of about 0.0005 g/L to about 1.000 g/L, or about 0.005 g/L to about 1.000 g/L, or about 0.010 g/L to about 0.100 g/L, or about 0.025 g/L to about 0.075 g/L, or about 0.040 g/L to about 0.060 g/L. In one embodiment, R1 comprises MnCl₂.4H₂O as a catalyst in a concentration of about 0.0525 g/L. The MnCl₂.4H₂O may serve additional functions beyond its catalytic properties, for instance, it is believed that MnCl₂.4H₂O may also act as an enzyme stabilizer to thereby improve the shelf-life of the enzyme reagent (R1).

In one embodiment, R1 comprises at least one protein stabilizer. A protein stabilizer will aid in the stabilization of the enzyme present in the reagent, thereby improving the shelf-life of the reagent. Any suitable protein stabilizer or combination thereof may be utilized in accordance with the invention.

One preferred protein stabilizer is PVP-40, which may also serve as a protein solubilizer in the reagent. The present inventors have found that PVP-40 can reduce or eliminate measurement errors in the assay caused by the presence of protein in the reagent and can prevent precipitation of protein in the reagent, thereby improving the shelf-life of the reagent and the overall performance of the assay. In one embodiment, R1 comprises PVP-40 in a concentration of about 0.1 g/L to about 10 g/L, or about 0.5 g/L to about 5 g/L, or about 1 g/L to about 3 g/L. In one embodiment, R1 comprises PVP-40 in a concentration of about 2 g/L.

In one embodiment, R1 comprises at least one protein stabilizer selected from PVP-40, BSA Fraction V, trehalose, sodium p-hydroxybenzoate, p-hydroxybenzoic acid or a combination thereof. In one embodiment, the at least one enzyme stabilizer comprises a combination of PVP-40, BSA Fraction V, trehalose and sodium p-hydroxybenzoate or p-hydroxybenzoic acid. The BSA Fraction V may be present in a concentration of, for example, about 0.1 g/L to about 10 g/L, or about 0.5 g/L to about 5 g/L, or about 1 g/L to about 2.5 g/L.

In one embodiment, R1 comprises BSA Fraction V in a concentration of about 1 g/L. Trehalose may be present in a concentration of, for example, about 0.1 g/L to about 10 g/L, or about 0.5 g/L to about 5 g/L, or about 1 g/L to about 2.5 g/L. In one embodiment, R1 comprises trehalose in a concentration of about 4.04 g/L. The p-hydroxybenzoic acid or p-hydroxybenzoic acid may be present in a concentration of, for example, about 0.1 g/L to about 10 g/L, or about 0.5 g/L to about 5 g/L, or about 1 g/L to about 2.5 g/L. In one embodiment, R1 comprises sodium p-hydroxybenzoate in a concentration of about 1 g/L. In one embodiment, R1 comprises p-hydroxybenzoic acid in a concentration of about 1 g/L.

In one embodiment, R1 comprises about 2 g/L PVP-40; about 1 g/L BSA Fraction V; about 4.04 g/L trehalose; and about 1 g/l sodium p-hydroxybenzoic acid.

R1 may optionally comprise a buffer. Any suitable buffer may be utilized in accordance with the invention. Suitable buffers may include, but are not limited to, phosphate, pyrophosphate, potassium phosphate, CAPS (N-Cyclohexyl-3-aminopropane sulfonic acid), CAPS/Metaborate, CAPS/Carbonate, tris(hydroxymethyl)aminomethane (TRIS), 2{[tris(hydroxymethyl)methyl]amino}-1-ethanesulfonic acid (TES), TRIS/Carbonate, 4-(2-hydroxyethyl)-1-piperazineethanesulfonic acid (HEPES), 3-[4-(2-hydroxyethyl)-1-piperazinyl]propanesulfonic acid (EPPES), 2-hydroxy-3-{N-[tris(hydroxymethyl)methyl]amino}-propanesulfonic acid (TAPSO), and combinations thereof.

The buffer may be present, for example, in a concentration of about 1 to 10 g/L or about 5 to 8 g/L. In one embodiment, R1 comprises CAPS buffer. In one embodiment, R1 comprises CAPS buffer in a concentration of about 6.4 or 6.5 g/L.

R1 may optionally comprise a preservative. Any suitable preservative may be utilized in accordance with the invention. Suitable preservatives include, but are not limited to, gentamycin sulfate, sodium azide, and sodium benzoate. In one embodiment, R1 comprises gentamycin sulfate in a concentration of about 0.001 g/L to about 0.1 g/L, or about 0.01 g/L to about 0.05 g/L. In one embodiment, R1 comprises about 0.01 g/L gentamycin sulfate. In one embodiment, R1 comprises sodium azide in a concentration of about 0.001 g/L to about 0.1 g/L, or about 0.01 g/L to about 0.05 g/L. In one embodiment, R1 comprises about 0.05 g/L sodium azide. In one embodiment, R1 comprises about 0.01 g/L gentamycin sulfate and about 0.05 g/L sodium azide.

R1 may optionally comprise a metal chelator. Any suitable metal chelator may be utilized in accordance with the invention. Suitable metal chelators include, but are not limited to, EDTA. In one embodiment, R1 comprises EDTA in a concentration of about 0.001 g/L to about 0.1 g/L, or about 0.01 g/L to about 0.05 g/L. In one embodiment, R1 comprises EDTA in a concentration of about 0.025 g/L.

R1 may optionally comprise a surfactant. Any suitable surfactant may be utilized in accordance with the invention. Exemplary surfactants include, but are not limited to, Brij^{™}-35, Triton^{™} X-100, Olin-10G^{™}, TX^{™}-102, TX-405^{™}, Zonyl FSN^{™}, TX-100^{™}, and TX-165^{™}.

The hydrolysis reaction preferably takes place at a pH in the range of about 5.9 to about 12.0, or about 6.5 to about 9.0, or about 7.5 to about 9.4, preferably about 8 to 9. In one embodiment, the pH is about 8.6.

The pH of R1 may be adjusted by any suitable means known in the art. For example, NaOH or any other suitable base may be used to increase pH. HCl or any other suitable acid may be used to decrease pH. In one embodiment, the pH of R1 is adjusted using NaOH. In one embodiment, R1 comprises 2N NaOH in an amount of about 500 µL/L to about 1000 µL/L. In one embodiment, R1 comprises about 833 µL/L 2N NaOH.

An exemplary R1 formulation is provided in Table 1 below. In accordance with this exemplary embodiment, R1 may be prepared by adding each of the components, with the exception of the enzyme and sodium azide, to less than 100% total volume of a suitable diluent, preferably distilled water, deionized water or reverse osmosis water. The pH is then adjusted to the desired range with NaOH, followed by addition of the sodium azide. The enzyme is added last. The formulation is then made up to 100% volume with the diluent.

In one embodiment of an automated assay of the present invention, 10 µL of sample (or control or standard) is added to 100 µL of R1 in a cuvette. The R1 is then subjected to a 1:1 on board dilution with 100 µL water, preferably deionized water, distilled water or reverse osmosis water, and the solution is mixed briefly. The hydrolysis reaction takes place in the cuvette. Volumes including but not limited to volumes up to 50 µl, 100 µl, 200 µl, 210 µl, 250 µl, 300 µl, 400 µl, and 500 µl, and higher, may be adjusted depending on the size of the cuvette required for a particular automated instrument (i.e. chemical analyzer). In one embodiment, the chemical analyzer is a Hitachi 717^{®} Chemical Analyzer (Roche Diagnostics).

The hydrolysis reaction may take place at a temperature of about 10° C. to about 60° C., or about 30° C. to about 50° C., or about 35° C. to about 40° C. In one embodiment, the hydrolysis reaction takes place at a temperature of about 37° C.

The hydrolysis reaction is allowed to proceed for a sufficient amount of time to permit hydrolysis of substantially all of the acetaminophen present in the sample (or standard), typically between about 2 to 20 minutes or between about 3 to 10 minutes. In one embodiment, the hydrolysis reaction is continued for about 5 minutes. The length of the reaction can be optimized for the selected temperature since longer reaction times are generally needed for lower temperatures.

After the hydrolysis reaction is complete and substantially all of the acetaminophen in the sample (or standard) has been converted to p-aminophenol, the oxidative coupling step is carried out.

In one embodiment of an automated assay of the present invention, the oxidative coupling step is initiated by adding the second reagent (R2) containing the chromophore, where optionally the chromophore has been pre-dissolved in DMSO, directly to the cuvette containing the hydrolysis solution. In accordance with the embodiment described above, 200 µL R2 is added to the hydrolysis solution, and is mixed briefly, for a final oxidative coupling reaction volume of 410 µL (10 µL sample+100 µL R1+100 µL water+200 µL R2) in the cuvette.

The preferred chromophore is a xylenol chromophore. Bulger et al (US 8,236,519 B2) disclose that the choice of a xylenol chromophore in the second reagent (R2) of their two step assay significantly reduced interference in the presence of NAC. The significantly reduced interference in the presence of NAC allows the use of their assay to analyze samples from patients undergoing-NAC treatment. Bulger et al (US 8,236,519 B2) disclose reliable acetaminophen measurements in the presence of therapeutic levels of NAC up to at least 2000 mg/L.

It is well known in the art that the presence of lipid molecules (Lipemia) is one of the most common pre-analytical interferences, the interference resulting from sample turbidity caused by accumulation of the lipoprotein particles. Lipemia can cause increased absorption of light, affecting tests that use spectrophotometric methods. The amount of absorbed light is inversely proportional to the wavelength and decreases from 300 to 700 nm, with no specific absorption peaks in between. Therefore, assays and methods that use spectrometric methods for detecting lower wavelengths are affected by lipemia, because the absorbance is the highest in that part of the spectra. Absorbance is proportional to the amount of lipids in the sample.

Typically, the prior art recommends the removal of lipids before treating lipemia samples, e.g., by ultracentrifugation, extraction using polar solvents or sample dilution. Each of these protocols has disadvantages, e.g., the high cost of ultracentrifuges and decrease in sensitivity of assay.

The present inventors have surprisingly discovered that the addition of DMSO to the second reagent (R2) containing the xylenol chromophore in the two step assay for acetaminophen disclosed by Bulger et al. significantly reduced interference in the assay due to the presence of lipid molecules in the NAC containing sample.

Thus, this surprising effect of DMSO in reducing lipemia interference in the acetaminophen assay by adding DMSO to R2 provides an advantage over prior art assays in that the present assay not only can be recommended for use on samples from patients undergoing NAC treatment, it can be used with samples containing lipid molecules, including but not limited to a concentration of up to approximately 3000 mg/dL triglycerides, without the need to remove the lipid molecules.

The present inventors have surprisingly discovered that the addition of DMSO to the second reagent (R2) containing the xylenol chromophore in the two step assay for acetaminophen disclosed by Bulger et al. also increases the sensitivity of the assay of samples containing NAC.

These surprising results provide advantages over prior art assays because the present assay can be recommended for use on lipemic samples from patients undergoing during NAC treatment. Further, practitioners need not be as concerned with variability in the content of lipid molecules in samples obtained from a patient over time. It is well established in the art that the lipemia value of a patient sample is in part dependent on timing and content of the patient's last meal.

Additional causes of lipemia samples are well known in the art including samples from patients having various primary and secondary disorders such as Fredrickson type I, IV, or V hyperlipidemia, diabetes mellitus, alcoholism, renal disease, nonalcoholic fatty liver disorder and HIV infection, or as the result of taking certain medications.

The use of a xylenol chromophore in accordance with the present invention, 2,5-dimethylphenol, 2,6-dimethylphenol, or 2,3-dimethylphenol, results in minimal interference in the assay in the presence of therapeutic levels of NAC. In experiments, 2,5-dimethylphenol, 2,6-dimethylphenol or 2,3-dimethylphenol showed no significant interference with 1471 mg/L NAC in an interference assay. In one embodiment, the xylenol chromophore is 2,5-dimethylphenol (p-xylenol).

The xylenol chromophore may be present during the oxidative coupling reaction in any suitable concentration. In order to accurately calculate the acetaminophen concentration present in the initial sample however, the chromophore should ideally be present in a molar concentration that meets or exceeds the maximum p-aminophenol molar concentration in the hydrolysis solution, which is proportional to the amount of acetaminophen in the initial sample.

In one embodiment, R2 comprises the xylenol chromophore in a concentration of about 0.075 g/L to about 115 g/L, or about 2.5 g/L to about 20 g/L, or about 5 g/L to about 10 g/L. In one embodiment, the xylenol chromophore is 2,5-dimethylphenol and is present in R2 in a concentration of about 7.5 g/L. In a preferred embodiment, the chromophore is pre-dissolved in DMSO before being added to the remaining components of R2. In a preferred embodiment, the chromophore pre-dissolved in DMSO is 2,5-dimethylphenol.

In order for the oxidative coupling reaction to take place in a suitable timeframe, an appropriate catalyst must be present. The catalyst may be a component of R1 or R2, or may be added to the mixture of R1 and R2. A variety of catalysts can be used in accordance with the present invention, such as permanganates, periodates, persulfates, and various metal salts. In one embodiment, the catalyst is a metal salt, such as FeCl₃, MnCl₂, CuSO₄, or KIO₄. In a preferred embodiment, the catalyst comprises anhydrous or hydrated MnCl₂. In a preferred embodiment, MnCl₂, which is a weak oxidizer, was particularly effective in catalyzing the oxidative coupling step with p-xylenol compared to other metal salts tested.

Typically, for the oxidative coupling step in an acetaminophen assay, a catalyst having a strong oxidizing potential is selected to ensure enough energy is provided to drive the coupling reaction to completion. The catalysts generally employed are metal salts of species containing reactive oxygen or functional groups, such as sodium periodate, copper sulfate and manganese acetate. For instance, known acetaminophen assays have utilized hydroxyquinoline or its derivatives catalyzed by manganese acetate (i.e. Sekisui Diagnostics P.E.I. Inc., PEI, Canada); o-cresol catalyzed by periodate (GDS Diagnostics) or copper sulfate (Hammond et al., 1984); or p-xylenol catalyzed by sodium periodate (Afshari and Lui, 2001). Such acetaminophen assays are known to show interference. For instance, interference in the presence of elevated levels of bilirubin in biological samples producing erroneous results in such samples.

Bulger et al disclosed that the use of a strong oxidizing catalyst could be a contributing factor in the bilirubin interference that is seen with conventional acetaminophen assays. For instance, periodate has been used by certain reagent manufacturers to measure bilirubin by selective destruction of the bilirubin to form bilverdin. There is a significant change in absorbance as bilirubin is oxidized to bilverdin or higher oxidative products, which could contribute to the interference seen in conventional assays. Bulger et al disclosed that (a) a weak oxidizer could successfully drive an oxidative coupling reaction between a xylenol chromophore, such as 2,5-dimethylphenol, and p-aminophenol and (b) the use of a weak oxidizer would have a positive effect on bilirubin interference in the assay.

Specifically, Bulger et al disclosed that that the choice of a weak oxidizer, such as MnCl₂ as an exemplary catalyst having a low oxidative potential compared to sodium periodate or other strong oxidizers containing reactive oxygen species, provided sufficient energy to catalyze the coupling of p-aminophenol to 2,5-dimethylphenol in the reaction. Furthermore, Bulger et al disclosed that that the choice of a weak oxidizer as the catalyst significantly reduced bilirubin interference in the assay, which is a highly desirable finding from a clinical perspective. Bulger et al disclosed further that a lower concentration of MnCl₂ was needed to drive the reaction compared to other catalysts tested and yet a stronger color development occurred in the assay. Bulger et al disclosed that the use of less catalyst in the reagent reduces the chance of the catalyst reacting with other reagent components or with biological or chemical components present in the patient samples, thereby improving the assay.

The choice of a weak oxidizer as a catalyst may therefore reduce the potential for spontaneous oxidation of the xylenol chromophore and reactivity with other components present in the assay reagent over time, thereby improving reagent stability and extending the shelf-life of liquid-stable reagents. A skilled person will be able to distinguish a strong oxidizer from a weak oxidizer and will be able to select a suitable weak oxidizer for use as a catalyst in accordance with embodiments of the invention.

Bulger et al disclosed that the addition of an antioxidant to the chromophore reagent could further improve the color stability of the reagent over time. Specifically, Bulger et al disclosed that addition of reduced glutathione, a component not commonly found in chromogenic assays, successfully prevented color development in the chromophore reagent over time, likely due in part to prevention of xylenol auto-oxidation, thereby improving reagent stability. Glutathione also functions as a scavenger and thus may remove radicals in the reagent that could potentially interfere in the oxidative coupling reaction.

Bulger et al disclosed conducting studies using hydroxylamine, 3,3'-thiodipropionic acid, thiourea or reduced glutathione, where the reagents were monitored both qualitatively and quantitatively for color change over time. Bulger et al disclosed that the addition of reduced glutathione to the reagent was the most effective in preventing color development over time.

Thus, R2 may optionally contain an anti-oxidant. Any suitable antioxidant may be utilized in accordance with the present invention. In one embodiment, R2 comprises an antioxidant in a concentration of about 0.005 g/L to about 5.00 g/L, or about 0.05 g/L to about 5 g/L, or about 0.1 g/L to about 1 g/L. In a preferred embodiment, the antioxidant is glutathione. Reduced glutathione is particularly preferred. In one embodiment, R2 comprises reduced glutathione in a concentration of about 0.5 g/L.

R2 may optionally contain one or more of a buffer or a surfactant or a combination thereof. Any suitable buffer or surfactant may be utilized in accordance with the present invention, for example, those mentioned above with respect to the hydrolysis solution. The presence of a surfactant in the reagent may inhibit lipemic interference in the assay, particularly at low acetaminophen levels (i.e. <200 µmol/L).

In one embodiment, R2 comprises TRIS in a concentration of about 10 to 50 g/L or about 15 to 30 g/L or about 20 to 30 g/L. In one embodiment, R2 comprises about 24.2 g/L TRIS. In one embodiment, R2 comprises sodium carbonate in a concentration of about 5 to 20 g/L or about 10 to 15 g/L. In one embodiment, R2 comprises about 10.6 g/L sodium carbonate. In one embodiment, R2 comprises about 24.2 g/L TRIS and about 10.6 g/L sodium carbonate.

In some embodiments, the coupling reaction is carried out at about 37° C. with a basic pH between about 9 and 12, or between about 9.5 and 11.5, or preferably between about 10 and 11. In one embodiment, the pH is greater than about 10. In one embodiment, the pH is about 10.8.

The pH of the reagent may be adjusted by any suitable means known in the art. In one embodiment, NaOH pellets are added to R2 in a concentration of about 1 g/L to about 4 g/L or about 2 g/L to about 3 g/L. In one embodiment, R2 comprises about 2.5 g/L NaOH pellets.

An exemplary R2 formulation disclosed by Bulger et al is shown in Table 2 below. In preparing R2 in accordance with this exemplary embodiment, it is recommended that the glutathione and 2,5-dimethylphenol be added last and in that order.

The pH of the assay reagent may be checked after preparation and can be further adjusted if needed.

The oxidative coupling reaction may take place at a temperature of about 10° C. to about 60° C., or about 30° C. to about 50° C., or about 35° C. to about 40° C. In one preferred embodiment, the oxidative coupling reaction takes place at a temperature of about 37° C.

The oxidative coupling reaction is allowed to proceed for a sufficient amount of time to permit coupling of the xylenol chromophore with substantially all of the p-aminophenol present in the reaction mixture, typically, but not limited to between about 2 to 20 minutes or between about 3 to 10 minutes. In one embodiment, the oxidative coupling reaction is continued for about 5 minutes. The length of the reaction can be optimized for the selected temperature since longer reaction times are generally needed for lower temperatures.

The oxidative coupling of the xylenol chromophore with p-aminophenol results in the formation of a blue product (i.e. a dye), which may be detected by measuring the change in absorbance of the assay mixture at an appropriate wavelength. The absorbance at the end of the hydrolysis reaction is subtracted from the absorbance at the end of the oxidative coupling reaction. The stoichiometric amount of acetaminophen present in the original sample is substantially equivalent to the stoichiometric amount of dye formed.

The absorbance of the resulting dye can be measured over a range of wavelengths. The maximum absorbance of the dye typically occurs at about 610-615 nm. Typically, the wavelength selected for measurement in a colorimetric assay is the wavelength at which peak absorbance occurs. If a bichromatic analyzer is utilized, a bichromatic blanking measurement is taken at an alternate wavelength, such as including but not limited to about 700 nm to about 850 nm, which is subtracted from the primary measurement to minimize background noise in the assay. Other known methods of minimizing background noise in an assay may also be used.

Bulger et al disclose that measuring the absorbance at an off-peak wavelength (i.e. on the shoulder of the absorbance curve rather than the peak) significantly decreased interference with the biological molecules bilirubin and hemoglobin in the assay. It was found that measuring absorbance at a wavelength of about 640 nm to about 680 nm, or about 650 nm to 670 nm, preferably about 660 nm, significantly improved acetaminophen measurement accuracy in the presence of bilirubin or hemoglobin. Bilirubin and hemoglobin interference are common disadvantages associated with known acetaminophen assays. Thus, to minimize interference with in the assay, the absorbance may be measured at 640 nm to about 680 nm, or about 650 nm to 670 nm, preferably about 660 nm, but is not limited to these wavelengths.

In one embodiment, absorbance is measured between about 610 and 665 nm.

A skilled person will appreciate that while the components and their relative proportions in the reagent solutions can be varied without departing from the present invention, the presence of cloudiness, precipitates and other contaminating factors are to be avoided, both in the individual and combined solutions. Where the assay is a liquid-stable assay, any alterations that could negatively impact the stability of the reagents or the components thereof, such as enzyme or chromophore stability, should be assessed carefully.

In one exemplary embodiment, a two-part acetaminophen assay in accordance with the present invention is carried out as summarized briefly below.

The first part consists of the addition of an enzyme reagent (R1) to a patient serum or plasma sample at a certain sample to reagent ratio in a cuvette. On the Hitachi 717, for example, the sample volume is 10 µL and the R1 volume is 100 µL with a 100 µL on board dilution of R1 with de-ionized water to form a hydrolysis solution. The solution is allowed to incubate at 37° C. for set duration of time, such as 5 minutes, on the automated analyzer. During this time, the aryl acylamidase enzyme present in the reagent cleaves the amide bond of the acetaminophen molecule in the sample leaving p-aminophenol and acetate. Absorbance readings are monitored at an established wavelength and at certain time intervals prior to the introduction of a chromophore reagent.

In the second part, the chromophore reagent (R2) is introduced at a set time interval and a certain volume to the hydrolysis solution (sample+R1, diluted). On the Hitachi 717, for example, 200 µL of R2 is introduced and the reaction is monitored at set time intervals until completion of the time duration for the test. The xylenol chromophore in R2, which preferably is 2,5-dimethylphenol, preferably is pre-dissolved in DMSO before being added to the remaining components of R2, oxidatively couples at an alkaline pH, in the presence of catalyst, with the p-aminophenol produced in the first part. The 2,5-dimethylphenol is present at 25x excess, up to 22.5x, up to 20x, up to 17.5x, up to 15x, up to 12.5x, up to 10x, or up to 5x excess, preferably 12.5x excess. In one preferred embodiment, the oxidative coupling step is carried out in the presence of manganese cations. The reaction produces a colored complex that has a maximal absorption peak at about 610 nm.

The analyzer takes the difference between the absorbance prior to R2 addition and the absorbance at the end of the reaction, corrected for background noise. The difference in optical density is the amount of absorbance produced from that sample. The change in absorbance can be compared against a standard curve to calculate acetaminophen concentration in the original sample.

In a preferred embodiment, the absorbance is measured at 660 nm to minimize interference with certain biological molecules in the assay.

While not wishing to be bound by any particular theory, it is believed that the concentration of acetaminophen is directly proportional to the intensity of the absorbance according to the principle known as the Beer-Lambert Law, A=εcl, where:
A=absorbance (at a given wavelength);
ε=molar extinction coefficient (a constant for every chemical);
l=length of the light path (i.e. 1 cm); and
c=concentration of the solute.

Therefore, the concentration of the solute (in this case acetaminophen) is directly proportional to absorbance given that the molar extinction coefficient and the path length is constant.

In one embodiment, the concentrations in the final reaction mixture are as follows, where the 2,5-dimethylphenol is dissolved in DMSO.
1) 227.5 U/L Aryl Acylamidase
2) 0.0128 g/L MnCl₂
3) 1.83 g/L 2,5-dimethylphenol
4) 0.244 g/L Glutathione

The assay of the present invention may be produced and sold as a kit of parts. The reagents in the kit may be powdered or lyophilized reagents requiring reconstitution. Methods of making such powdered or lyophilized reagents are known in the art. Preferably, the reagents are liquid-stable reagents. Liquid stable reagents are convenient to use and are less prone to errors that can be introduced during reconstitution.

In one embodiment, the kit comprises: a vessel comprising an enzyme reagent (R1); a vessel comprising a chromophore reagent (R2); and optionally directions for carrying out the assay specifying use of DMSO to dissolve the chromophore. The kit may further comprise an acetaminophen standard and directions for preparing a linear set of standards.

### WORKING EXAMPLES

### Example 1

Tables 1 and 2 provide Exemplary Enzyme and Chromophore Reagents disclosed in Bulger et al.

**TABLE 1**

| Exemplary enzyme reagent (R1) composition with deionized water as diluent. | | | | |
|---|---|---|---|---|
| Ingredient | Amount/ L | Amount/ 100 µL | Conc. in Final Volume of 410 µL (10 µL sample + 100 µL R1 + 100 µL H₂O + 200 µL R2) | Primary Purpose (may serve other functions as well) |
| CAPS | 6.460 g | 646 µg | 158 µg | Buffer |
| MnCl2·4H2O | 0.0525 g | 5.25 µg | 1.31 µg | Catalyst |
| BSA | 1.000 g | 100 µg | 24.4 µg | Enzyme |
| Fraction V Trehalose | 4.040 g | 404 µg | 98.9 µg | Stabilizer Enzyme |

**TABLE 1-continued**

| Exemplary enzyme reagent (R1) composition with deionized water as diluent. | | | | |
|---|---|---|---|---|
| Ingredient | Amount/ L | Amount/ 100 µL | Conc. in Final Volume of 410 µL (10 µL sample + 100 µL R1 + 100 µL H₂O + 200 µL R2) | Primary Purpose (may serve other functions as well) |
| Gentamycin Sulfate | 0.010 g | 1.0 µg | 2.44 µg | Stabilizer Preservative |
| Sodium p-Hydroxy-benzoate | 1.000 g | 100 µg | 24.4 µg | Enzyme Stabilizer |
| EDTA | 0.025 g | 2.5 µg | 0.61 µg | Metal Chelator |
| PVP-40 | 2.000 g | 200 µg | 48.8 µg | Protein Stabilizer |
| 2N NaOH | 833 µL | 0.083 µL | 0.02 µL | pH adjuster |
| Sodium Azide | 0.050 g | 5.0 µg | 1.2 µg | Preservative |
| Aryl Acylamidase | 932.7 U | 0.093 U | 0.023 U | Enzyme |

**TABLE 2**

| Exemplary chromophore reagent (R2) composition with deionized water as diluent. | | | | |
|---|---|---|---|---|
| Ingredient | Amount/ L | Amount/ 200µL | Conc. in Final Volume of 410 µL (101 µL sample + 100 µL R1 + 100 µL H2O +200 µL R2) | Primary Purpose (may serve other functions as well) |
| TRIS | 24.200 g | 4.840 mg | 2.360 mg | Buffer |
| Sodium Carbonate | 10.608 g | 2.121 mg | 1.061 mg | Buffer |
| NaOH Pellets | 2.500 g | 500 µg | 244 µg | Buffer |
| Glutathione | 0.500 g | 100 µg | 48.8 µg | Anti-oxidant |
| 2,5-Dimethylphenol | 7.500 g | 1.5 mg | 732 µg | Chromophore |

The reagents may be prepared in a suitable diluent, such as deionized water.

### Example 2

Performance of Assay in Absence and Presence of NAC using the Exemplary Enzyme and Chromophore Reagents disclosed in Tables 1 and 2.

A set of standards was prepared having a known concentration of acetaminophen in deionized water (i.e. 250 µmol/L, 1000 µmol/L, 1500 µmol/L, 2000 µmol/L, and 2500 µmol/L). A 10 µL aliquot of each standard was added to 100 µL of R1 in a cuvette in a Hitachi 717^{®} Analyzer (Roche Diagnostics) followed by an on board dilution with 100 µL deionized water. Each mixture was allowed to incubate at 37° C. for 5 minutes. An initial absorbance value was obtained for each. A 200 µL aliquot of R2 was added to each cuvette. The oxidative-coupling reaction was allowed to proceed for 5 minutes. A final absorbance value was obtained. The concentration of acetaminophen was calculated based on the difference in absorbance at 660 nm between the final and initial absorbance, corrected for background noise by subtracting the absorbance at 800 nm. The results for various known concentrations are shown in Table 3 below.

**TABLE 3**

| Measured acetaminophen concentration in the absence of NAC using p-xylenol as chromophore. | | |
|---|---|---|
| [Acetaminophen] in sample (µmol/L) | Measured [Acetaminophen] (µmol/L) | % Difference |
| 250 | 248 | -0.8 |
| 500 | 496 | -0.8 |
| 1000 | 995 | -0.5 |
| 1500 | 1498 | -0.1 |
| 2000 | 1987 | -0.7 |
| 2500 | 2479 | -0.8 |

Linearity assessment was conducted using prepared serum-based linearity material. Sample concentrations were evaluated up to 3000umol/L.

| Theoretical (µmol/L) | Measured (µmol/L) | % Variance |
|---|---|---|
| 250 | 248.5 | -0.6 |
| 500 | 504.8 | 1.0 |
| 1000 | 1013.5 | 1.3 |
| 1500 | 1522.0 | 1.5 |
| 2000 | 2040.0 | 2.0 |
| 2500 | 2525.3 | 1.0 |
| 3000 | 3022.3 | 0.7 |

The assay demonstrated accurate measurement of acetaminophen concentration across a wide range of acetaminophen concentrations.

To assess performance in the presence of NAC, a stock solution was made by dissolving 75 mg of NAC into 1000 µL of deionized water. This produced a 75 g/L or 75000 mg/L NAC concentrated stock solution.

A 2.5 ml aliquot of a known concentration of acetaminophen in water was added to a test tube and this was spiked with 50 µL of the NAC stock solution to prepare a set of spiked standards having a known concentration of acetaminophen (i.e. 245 µmol/L, 490 µmol/L, 980 µmol/L, 1470 µmol/L, 1960 µmol/L, and 2450 µmol/L). Each spiked acetaminophen standard had a NAC concentration of 1471 mg/L, which is a value that could be found in patient serum during NAC treatment. Samples were analyzed within an hour of spiking since NAC degrades over time.

The assay was carried out as described immediately above. A 10 µL aliquot of spiked standard was added to 100 µL of R1 in a cuvette in a Hitachi 717^{®} followed by an on board dilution. Each mixture was allowed to incubate at 37° C. for 5 minutes. An initial absorbance value was obtained. A 200 µL aliquot of R2 was added to the respective cuvettes. The oxidative-coupling reaction was allowed to proceed for 5 minutes. Based on the difference in absorbance at 660 nm, corrected for background, the concentrations of acetaminophen were calculated. Method comparison was done on the Siemens Advia^{®} 1650 to compare a known assay using an 8-HQ derivative as the chromophore. The results for various known concentrations of acetaminophen are shown in Table 4 below.

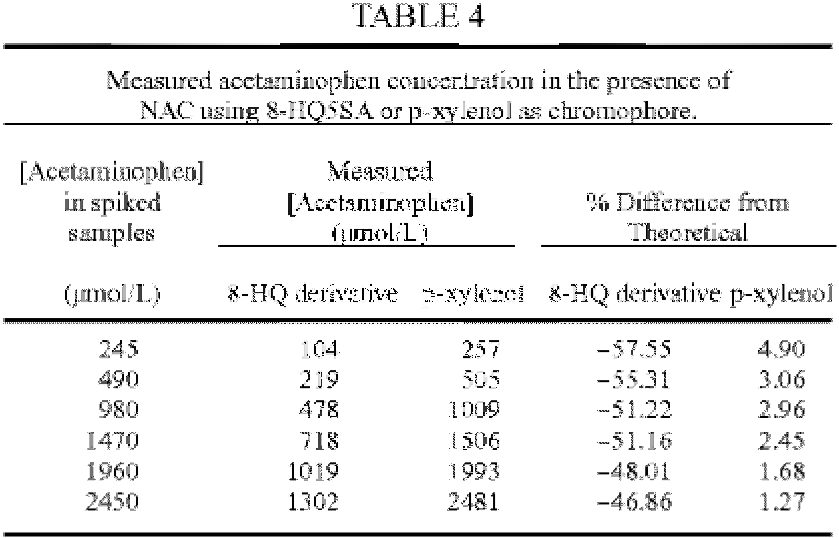

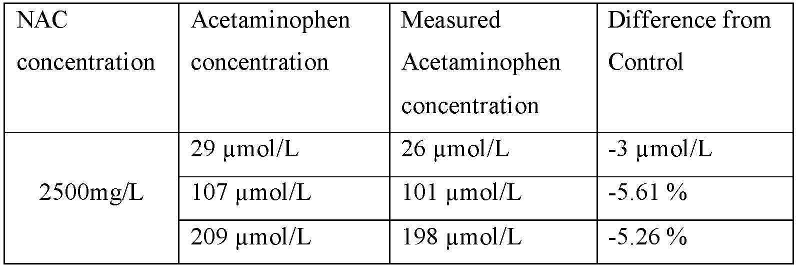

The assay with p-xylenol as the chromophore was resistant to interference in the presence of NAC compared to an assay using an 8-HQ derivative as the chromophore. The cut off for interference in a clinical assay is generally a 10% difference, preferably less than 5%.

### Example 3

### Comparison of Xylenol Chromophores

Comparison of the xylenol chromophores was conducted by substituting the chromophore in the R2 formulation of Working Example 1. All other parameters were the same. A stock solution of the R2 buffer and glutathione was made and then split into six batches. 7.5 g/L, of each respective isomer was dissolved in each respective batch, thereby creating six "different" chromophore reagents with a different isomer in each, R1 was kept constant therefore the only variable in the analysis was the chromophore in R2, The enzyme reagent (R1) had a pH of 8.6 @ 25° C., while the six isomeric xylenol reagents each had a pH of 11.5@ 25° C. The results of the various known isomers are shown in Table 5 below.

The results demonstrated that 2,5-dimethylphenol, 2,6-dimethylphenol, and 2,3-dimethylphenol produced acceptable linearity results in the presence of NAC. The best performance was seen with 2,5-dimethylphenol and 2,6-dimethylphenol.

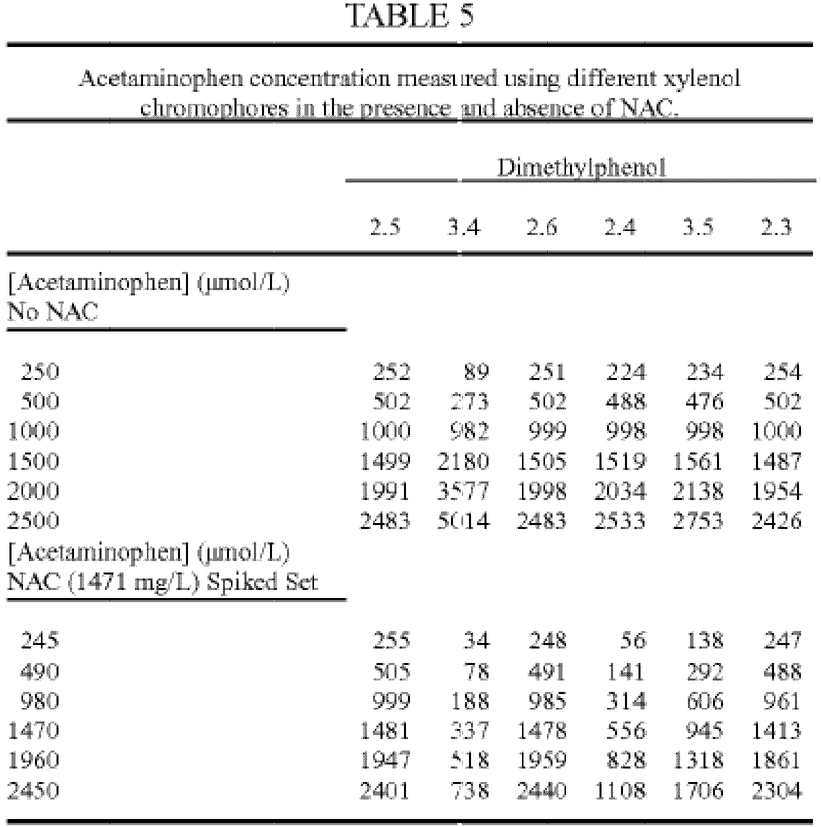

### Example 4 Evaluation of 2,5-dimethylphenol in the Presence of Intralipid^{®}

An evaluation of 2,5-dimethylphenol was carried out using Intralipid^{®}-spiked serum containing a known concentration of acetaminophen. It was shown that, at a therapeutic level of acetaminophen (<200 µmol/L), the 2,5-dimethylphenol chromophore recovered within the acceptable limit of ±10% at 200 mg/dL Intralipid^{®}. Testing was carried out on the Siemens Advia^{®} 1650.

INTRALIPID^{®} 20% is a sterile, non-pyrogenic fat emulsion prepared for intravenous administration as a source of calories and essential fatty acids, it is made up of 20% soybean oil, 1.2% egg yolk phospholipids, 2.25% glycerin and water for injection" according to the web site https://www.rxlist.com/intralipid-20-drug.htm

First, 10 ml of serum was pooled into a large test tube and mixed. The serum was then spiked with 2.1 mg of reagent grade acetaminophen and mixed until dissolved. From the spiked pool, one 4.75 ml aliquot was pipetted out and placed in a large test tube marked as control pool. A second aliquot of 4.75 ml was pipetted out and placed in a second large test tube marked as test pool. In the control pool, 250 µL of saline was added to the pool and mixed thoroughly. In the test pool, 250 µL of 20% Intralipid^{®} solution was added and mixed thoroughly. An interference set was made by mixing the control and test pools at varying levels, thereby creating different Intralipid^{®} concentrations but maintaining the acetaminophen concentration. The 2,5-dimethylphenol demonstrated acceptable results to at least 200 mg/dL Intralipid^{®}.

### Example 5 (Improved Assay)

A Reformulation of the Acetaminophen assay displayed in Tables 1 and 2 was designed to improve the assay's efficiency. In one aspect, the R2 Color Reagent manufacturing process of the Acetaminophen assay displayed in Table 2 was very slow, in large part because the chromophore was slow to dissolve. Accordingly, two changes were made to the Acetaminophen assay disclosed in Tables 1 and 2. First the chromophore 2,5-dimethylphenol was pre-dissolved in DMSO, before its addition to the remaining components of the chromophore reagent (R2). Second, the concentration of the chromophore 2,5-dimethylphenol in the chromophore reagent (R2) composition was cut in half. The 2,5-dimethylphenol was present at 24X excess in the formulation displayed in Tables 1 and 2. The concentration has been reduced to 12X excess in the reformulated assay.

The reformulated Acetaminophen assay is summarized below. Changes with respect to the assay presented in Tables 1 and 2 are underlined and highlighted in bold font.

**Table 6**

| Acetaminophen R1 | |
|---|---|
| ENZYME REAGENT | CONCENTRATION (g/L) |
| CAPS (3-Cyclohexylamino-1-propanesulfonic acid | 6.46 |
| Manganese Chloride.4H2O | 0.0525 |
| Albumin Bovine Fraction V Protease free | 1 |
| Trehalose (a,a-Trehalose, dihydrate | 4.04 |
| Gentamycin Sulfate (Potency-approximately 600 U/mg) | 0.01 |
| p-Hydroxybenzoic Acid Sodium Salt | 1 |
| EDTA - disodium 2H2O | 0.025 |
| Polyvinyl-pyrrolidone (PVP-40) | 2 |
| 2N NaOH. Adjust to pH 8.6 ± 0.1 | 0.833mL |
| Na Azide | 0.05 |
| 1N HCL (For pH adjustment) | As needed |
| Aryl Acylamide Amidohydrolase | 932.67U |

| Acetaminophen R2 | |
|---|---|
| COLOR REAGENT | CONCENTRATION |
| Tris, Free Base | 24.2 g/L |
| Na Carbonate Anhydrous | 10.61 g/L |
| NaOH | 2.75 g/L |
| Glutathione, reduced ~98% | 0.5 g/L |
| **DMSO (Dimethylsulfoxide)** | **5.5 g/L** |
| **2,5-dimethyl Phenol** | **3.75 g/L** |
| 2N NaOH, or IN HCl. Adjust to pH 11.53 - 11.60 | As needed |

The reformulated acetaminophen assay as presented in the above Table significantly improved the speed of the manufacturing process of the R2 Color Reagent. This improvement was effected by reducing in half the concentration of the 2,5-dimethylphenol chromophore and predissolving it in DMSO before adding it to the remaining components of the R2 Color Reagent,unexpectely improving its stability.

However, a completely unexpected property resulting from the reformulated acetaminophen assay as presented in the above Table was a significant improvement with respect to lipemia interference. The decrease in interference due to lipemia of the reformulated acetaminophen assay relative to the acetaminophen assay displayed in Tables 1 and 2 was demonstrated using Intralipid^{®}-spiked serum as described in working example 4.

In Example 4 the acetaminophen assay using the formulation of Tables 1 and 2 was able to detect 15.3 µg/mL acetaminophen in the presence of 200 mg/l of Intralipid^{®}. See Table 7 below. However, the reformulated acetaminophen assay as presented in the above Table 6 in which the chromophore 2,5-dimethylphenol was dissolved in DMSO and preset at a concentration of 12.5X excess, was able to detect 15.1 µg/mL acetaminophen in the presence of 1,000 mg/l of Intralipid^{®}, see Table 8 below, thus demonstrating a surprising and significant tolerance of lipemia by the reformulated assay as compared to the acetaminophen assay displayed in Tables 1 and 2. Other solvents were tested to improve the speed of the manufacturing process of the R2 Color Reagent by improving the 2,5-dimethylphenol chromophore solubility. Ethanol, methanol, and isopropyl alcohol were used successfully, but only DMSO was selected for further testing with lipemia interference.

**Table 7**

| Substance Tested | Concentration with no Significant Interference | Acetaminophen level |
|---|---|---|
| Intralipid | 200 mg/dL [600 mg/dL (6.8 mmol/L) Simulated Triglycerides]* | 15.3 µg/mL (101 µmol/L)* |

**Table 8**

| Substance Tested | Concentration With No Significant Interference | Acetaminophen Level |
|---|---|---|
| Intralipid | 600 mg/dL [1800 mg/dL (20 mmol/L) Simulated Triglycerides] | 4.8 µg/mL (32 µmol/L) |
| | 1000 mg/dL [3000 mg/dL (34 mmol/L) Simulated Triglycerides] | 15.1 µg/mL (100 µmol/L) |
| | 1000 mg/dL [3000 mg/dL (34 mmol/L) Simulated Triglycerides] | 30.7 µg/mL (203 µmol/L) |

### Example 6 Parameter Modifications Reduce Hemoglobin and Bilirubin Interference

Hemoglobin interference is a disadvantage of known acetaminophen assays. An Ultrospec^{™} 3300 scan of a 100 µmol/L acetaminophen sample spiked with 1000 mg/dL of hemoglobin was carried out and produced an interesting observation, The OD shift between a 100 µmol/L acetaminophen sample and the 100 µmol/L+1000 mg/dL Hemoglobin sample was much greater at 600 nm than at 660 nm, which was on the shoulder of the absorbance peak yet still provided a good OD shift between the primary and secondary wavelengths. Therefore further testing was conducted using 660 nm as the primary wavelength and keeping 800 nm as the secondary wavelength.

The results demonstrated that changing the primary wavelength from 600 nm to 660 nm significantly reduced interference in the presence of both hemoglobin and bilirubin in the assay. Analysis was carried out on both the Hitachi 717 and the Siemens Advia^{®} 1650. Serum was spiked with acetaminophen at a set concentration and then with the interfering material at varying concentrations. The modified assay showed acceptable levels of interference (i.e. <10%) in the presence of NAC, bilirubin and hemoglobin.

The above-described embodiments of the invention are intended to be examples only. Alterations, modifications and variations can be effected to the particular embodiments by those of skill in the art without departing from the scope of the invention, which is defined solely by the claims appended hereto.

## Claims

1. A method for determining the concentration of acetaminophen in an aqueous sample, the assay comprising the steps of
contacting the aqueous sample with a first reagent (RI) comprising an aryl acylamidase enzyme and a suitable diluent to form a hydrolysis solution, optionally, diluting the hydrolysis solution;
incubating the hydrolysis solution to permit a hydrolysis reaction wherein the acetaminophen is converted to p-aminophenol;
contacting the hydrolysis solution with a second reagent (R2) comprising a xylenol chromophore where the xylenol chromophore is pre-dissolved in DMSO, to form an oxidative coupling solution;
incubating the oxidative coupling solution to permit an oxidative coupling reaction wherein the xylenol chromophore is coupled to the p-aminophenol in the presence of a suitable catalyst to form a colored product; and
determining the amount of the colored product formed, the amount of the colored product formed being proportional to the amount of acetaminophen present in the aqueous sample,
wherein the xylenol chromophore is selected from the group consisting of 2,5-dimethylphenol, 2,6-dimethylphenol and 2,3-dimethylphenol and wherein the catalyst is a hydrated MnCl₂, and
wherein the method is reliable in the presence or absence of therapeutic levels of N-acetyl cysteine (NAC) in the aqueous sample.

2. The method of claim 1, wherein RI comprises aryl acylamidase at a concentration of about 10 U/L to about 5000 U/L.

3. The method of claim 2, wherein the xylenol chromophore is 2,5-dimethylphenol.

4. The method of claim 2, wherein the catalyst is present in RI in a concentration of about 0.0005 g/L to about 1.000 g/L.

5. The method of claim 4, wherein R2 comprises 2,5-dimethylphenol in a concentration of about 0.075 g/L to about 115 g/L and/or wherein the catalyst is MnCl₂.4H₂O and is present in R1 in a concentration of about 2.5 g/L to about 20 g/L.

6. The method of claim 4, wherein R2 further comprises reduced glutathione in a concentration of about 0.005 g/L to about 5.000 g/L.

7. The method of claim 6, wherein RI further comprises one or more of a protein solubilizer, a protein stabilizer, an enzyme stabilizer, a metal chelator, a buffer, a surfactant, a pH adjuster, a preservative, or an excipient.

8. The method of claim 7, wherein the enzyme stabilizer is selected from the group consisting of PVP-40, BSA Fraction V, trehalose, sodium p-hydroxybenzoate, p-hydroxybenzoic acid and combinations thereof.

9. The method of claim 8, wherein R2 further comprises one or more of a buffer, a surfactant, a pH adjuster, a preservative, an antioxidant or an excipient.

10. The method of claim 9, wherein the diluent is deionized water and the hydrolysis solution is diluted approximately 1:1 with the diluent prior to the hydrolysis reaction.

11. The method of claim 1, wherein RI comprises about 932.7 U/L aryl acylamidase and about 0.0525 g/L MnCl_{2.}4H2O; and wherein R2 comprises about 3.75 g/L 2,5-dimethylphenol and about 0.500 g/L reduced glutathione.

12. The method of claim 4, wherein the hydrolysis reaction and the oxidative coupling reaction each take place at a temperature of about 37° C. for about 3 to 10 minutes, and wherein the hydrolysis reaction takes place at a pH of about 8.6 and the oxidative coupling reaction takes place at a pH of about 10.8.

13. The method of claim 12, wherein the concentration of acetaminophen is determined by obtaining the difference in absorbance at the end of the hydrolysis reaction and at the end of the oxidative coupling reaction; and comparing the difference against a standard or set of standards, wherein the absorbance is measured at a wavelength between about 610 nm and 665 nm.

14. The method of claim 13, wherein the absorbance is measured at a wavelength of about 660 nm.

15. The method of claim 1, wherein the aqueous sample is serum or plasma.

16. A kit for determining the concentration of acetaminophen in a sample, the kit comprising:
a first reagent (R1) comprising an aryl acylamidase for hydrolyzing acetaminophen to p-aminophenol;
a second reagent (R2) comprising a xylenol chromophore for oxidative coupling to the p-aminophenol; and
a suitable catalyst for catalyzing the oxidative coupling of the xylenol chromophore and the p-aminophenol
wherein the xylenol chromophore is 2,5-dimethylphenol and the catalyst is hydrated MnC12,
and wherein R2 comprises 2,5-dimethylphenol dissolved in DMSO.

## Patentansprüche

1. Verfahren zum Bestimmen der Konzentration von Acetaminophen in einer wässrigen Probe, wobei der Test die folgenden Schritte umfasst:
Inkontaktbringen der wässrigen Probe mit einem ersten Reagenz (R1), das ein Aryl-Acylamidase-Enzym und ein geeignetes Verdünnungsmittel umfasst, um eine Hydrolyselösung zu bilden, gegebenenfalls Verdünnen der Hydrolyselösung;
Inkubieren der Hydrolyselösung, um eine Hydrolysereaktion zu ermöglichen, bei der das Acetaminophen in p-Aminophenol umgewandelt wird;
Inkontaktbringen der Hydrolyselösung mit einem zweiten Reagenz (R2), das einen Xylenol-Chromophor umfasst, wobei der Xylenol-Chromophor in DMSO vorgelöst ist, um eine oxidative Kopplungslösung zu bilden;
Inkubieren der oxidativen Kopplungslösung, um eine oxidative Kopplungsreaktion zu ermöglichen, bei der der Xylenol-Chromophorin in Gegenwart eines geeigneten Katalysators an das p-Aminophenol gekoppelt wird, um ein gefärbtes Produkt zu bilden; und
Bestimmen der Menge des gebildeten gefärbten Produkts, wobei die Menge des gebildeten gefärbten Produkts zu der Menge des in der wässrigen Probe vorliegenden Acetaminophens proportional ist,
wobei der Xylenol-Chromophor ausgewählt ist aus der Gruppe, bestehend aus 2,5-Dimethylphenol, 2,6-Dimethylphenol und 2,3-Dimethylphenol, und wobei der Katalysator ein hydratisiertes MnCl₂ ist, und
wobei das Verfahren bei Vorliegen oder Fehlen von therapeutischen Spiegeln von N-Acetylcystein (NAC) in der wässrigen Probe zuverlässig ist.

2. Verfahren nach Anspruch 1, wobei R1 Aryl-Acylamidase in einer Konzentration von etwa 10 U/L bis etwa 5000 U/L umfasst.

3. Verfahren nach Anspruch 2, wobei der Xylenol-Chromophor 2,5-Dimethylphenol ist.

4. Verfahren nach Anspruch 2, wobei der Katalysator in R1 in einer Konzentration von etwa 0,0005 g/L bis etwa 1,000 g/L vorliegt.

5. Verfahren nach Anspruch 4, wobei R2 2,5-Dimethylphenol in einer Konzentration von etwa 0,075 g/L bis etwa 115 g/L umfasst, und/oder wobei der Katalysator MnCl₂.4H₂O ist und in R1 in einer Konzentration von etwa 2,5 g/L bis etwa 20 g/L vorliegt.

6. Verfahren nach Anspruch 4, wobei R2 weiter reduziertes Glutathion in einer Konzentration von etwa 0,005 g/L bis etwa 5,000 g/L umfasst.

7. Verfahren nach Anspruch 6, wobei R1 weiter eines oder mehrere eines Proteinlösungsvermittlers, eines Proteinstabilisators, eines Enzymstabilisators, eines Metallchelators, eines Puffers, eines Tensids, eines pH-Einstellers, eines Konservierungsmittels oder eines Hilfsstoffs umfasst.

8. Verfahren nach Anspruch 7, wobei der Enzymstabilisator ausgewählt ist aus der Gruppe, bestehend aus PVP-40, BSA-Fraktion V, Trehalose, Natrium-p-hydroxybenzoat, p-Hydroxybenzoesäure und Kombinationen davon.

9. Verfahren nach Anspruch 8, wobei R2 weiter eines oder mehrere eines Puffers, eines Tensids, eines pH-Einstellers, eines Konservierungsmittels, eines Antioxidationsmittels oder eines Hilfsstoffs umfasst.

10. Verfahren nach Anspruch 9, wobei das Verdünnungsmittel deionisiertes Wasser ist und die Hydrolyselösung vor der Hydrolysereaktion ungefähr 1:1 mit dem Verdünnungsmittel verdünnt wird.

11. Verfahren nach Anspruch 1, wobei R1 etwa 932,7 U/L Aryl-Acylamidase und etwa 0,0525 g/L MnCl₂.4H₂O umfasst; und wobei R2 etwa 3,75 g/L 2,5-Dimethylphenol und etwa 0,500 g/L reduziertes Glutathion umfasst.

12. Verfahren nach Anspruch 4, wobei die Hydrolysereaktion und die oxidative Kopplungsreaktion jeweils bei einer Temperatur von etwa 37 °C über etwa 3 bis 10 Minuten stattfinden, und wobei die Hydrolysereaktion bei einem pH-Wert von etwa 8,6 stattfindet und die oxidative Kopplungsreaktion bei einem pH-Wert von etwa 10,8 stattfindet.

13. Verfahren nach Anspruch 12, wobei die Konzentration von Acetaminophen durch Erhalten der Absorptionsdifferenz am Ende der Hydrolysereaktion und am Ende der oxidativen Kopplungsreaktion; und Vergleichen der Differenz mit einem Standard oder einem Satz von Standards bestimmt wird, wobei die Absorption bei einer Wellenlänge zwischen etwa 610 nm und 665 nm gemessen wird.

14. Verfahren nach Anspruch 13, wobei die Absorption bei einer Wellenlänge von etwa 660 nm gemessen wird.

15. Verfahren nach Anspruch 1, wobei die wässrige Probe Serum oder Plasma ist.

16. Set zum Bestimmen der Konzentration von Acetaminophen in einer Probe, wobei das Set umfasst:
ein erstes Reagenz (R1), das eine Aryl-Acylamidase zum Hydrolysieren von Acetaminophen zu p-Aminophenol umfasst;
ein zweites Reagenz (R2), das einen Xylenol-Chromophor zum oxidativen Koppeln an das p-Aminophenol umfasst; und
einen geeigneten Katalysator zum Katalysieren der oxidativen Kopplung des Xylenol-Chromophors und des p-Aminophenols,
wobei der Xylenol-Chromophor 2,5-Dimethylphenol ist und der Katalysator hydratisiertes MnCl₂ ist,
und wobei R2 in DMSO gelöstes 2,5-Dimethylphenol umfasst.

## Revendications

1. Procédé de détermination de la concentration d'acétaminophène dans un échantillon aqueux, le dosage comprenant les étapes de
mise en contact de l'échantillon aqueux avec un premier réactif (R1) comprenant une enzyme aryl-acylamidase et un diluant approprié pour former une solution hydrolytique, éventuellement, dilution de la solution hydrolytique ;
incubation de la solution hydrolytique pour permettre une réaction d'hydrolyse, dans lequel l'acétaminophène est converti en p-aminophénol ;
mise en contact de la solution hydrolytique avec un second réactif (R2) comprenant un chromophore de xylénol, le chromophore de xylénol étant pré-dissous dans du DMSO, pour former une solution de couplage oxydatif ;
incubation de la solution de couplage oxydatif pour permettre une réaction de couplage oxydatif, dans lequel le chromophore de xylénol est couplé au p-aminophénol en présence d'un catalyseur approprié pour former un produit coloré ; et
détermination de la quantité du produit coloré formé, la quantité du produit coloré formé étant proportionnelle à la quantité d'acétaminophène présente dans l'échantillon aqueux,
dans lequel le chromophore de xylénol est choisi dans le groupe consistant en du 2,5-diméthylphénol, du 2,6-diméthylphénol et du 2,3-diméthylphénol, et dans lequel le catalyseur est un MnCL₂ hydraté, et
dans lequel le procédé est fiable en présence ou en l'absence de niveaux thérapeutiques de N-acétylcystéine (NAC) dans l'échantillon aqueux.

2. Procédé selon la revendication 1, dans lequel le R1 comprend de l'aryl-acylamidase en une concentration d'environ 10 U/L à environ 5 000 U/L.

3. Procédé selon la revendication 2, dans lequel le chromophore de xylénol est le 2,5-diméthylphénol.

4. Procédé selon la revendication 2, dans lequel le catalyseur est présent dans le R1 en une concentration d'environ 0,0005 g/L à environ 1,000 g/L.

5. Procédé selon la revendication 4, dans lequel le R2 comprend du 2,5-diméthylphénol en une concentration d'environ 0,075 g/L à environ 115 g/L et/ou dans lequel le catalyseur est MnCl₂.4H₂O et est présent dans le R1 en une concentration d'environ 2,5 g/L à environ 20 g/L.

6. Procédé selon la revendication 4, dans lequel le R2 comprend en outre du glutathion réduit en une concentration d'environ 0,005 g/L à environ 5,000 g/L.

7. Procédé selon la revendication 6, dans lequel le R1 comprend en outre un ou plusieurs parmi un solubilisant de protéine, un stabilisant de protéine, un stabilisant d'enzyme, un chélateur de métal, un tampon, un tensioactif, un ajusteur de pH, un conservateur ou un excipient.

8. Procédé selon la revendication 7, dans lequel le stabilisateur d'enzyme est choisi dans le groupe consistant en PVP-40, BSA Fraction V, tréhalose, p-hydroxybenzoate de sodium, acide p-hydroxybenzoïque et des combinaisons de ceux-ci.

9. Procédé selon la revendication 8, dans lequel le R2 comprend en outre un ou plusieurs parmi un tampon, un tensioactif, un ajusteur de pH, un conservateur, un antioxydant ou un excipient.

10. Procédé selon la revendication 9, dans lequel le diluant est de l'eau désionisée et la solution hydrolytique est diluée à approximativement 1:1 avec le diluant avant la réaction d'hydrolyse.

11. Procédé selon la revendication 1, dans lequel le R1 comprend environ 932,7 U/L d'aryl-acylamidase et environ 0,0525 g/L de MnCl₂.4H₂O ; et dans lequel le R2 comprend environ 3,75 g/L de 2,5-diméthylphénol et environ 0,500 g/L de glutathion réduit.

12. Procédé selon la revendication 4, dans lequel la réaction d'hydrolyse et la réaction de couplage oxydatif surviennent chacune à une température d'environ 37 °C pendant environ 3 à 10 minutes, et dans lequel la réaction d'hydrolyse survient à un pH d'environ 8,6 et la réaction de couplage oxydatif survient à un pH d'environ 10,8.

13. Procédé selon la revendication 12, dans lequel la concentration d'acétaminophène est déterminée en obtenant la différence d'absorbance à la fin de la réaction d'hydrolyse et à la fin de la réaction de couplage oxydatif ; et en comparant la différence à une norme ou à un ensemble de normes, dans lequel l'absorbance est mesurée à une longueur d'onde comprise entre environ 610 nm et 665 nm.

14. Procédé selon la revendication 13, dans lequel l'absorbance est mesurée à une longueur d'onde d'environ 660 nm.

15. Procédé selon la revendication 1, dans lequel l'échantillon aqueux est du sérum ou du plasma.

16. Kit pour déterminer la concentration d'acétaminophène dans un échantillon, le kit comprenant :
un premier réactif (R1) comprenant une aryl-acylamidase pour hydrolyser l'acétaminophène en p-aminophénol ;
un second réactif (R2) comprenant un chromophore de xylénol pour un couplage oxydatif au p-aminophénol ; et
un catalyseur approprié pour catalyser le couplage oxydatif du chromophore de xylénol et du p-aminophénol
dans lequel le chromophore de xylénol est du 2,5-diméthylphénol et le catalyseur est du MnCl2 hydraté,
et dans lequel le R2 comprend du 2,5-diméthylphénol dissous dans du DMSO.
